# FASCICULE DE BREVET EUROPEEN

(11) **EP 1 366 743 B1**
(45) Date de publication et mention de la délivrance du brevet: **05.07.2006**
(21) Numéro de dépôt: 03290972.3
(22) Date de dépôt: 18.04.2003
(51) Int. Cl.: A61K 8/63, A61Q 19/00

(54) **Utilisation d'un dérivé 7-oxydé de la DHEA pour le traitement des peaux sèches**
Verwendung von einem 7-oxydierten DHEA Derivat zur Behandlung trockener Haut
Use of a derivative of 7-oxydated DHEA for treating dry skin

(30) Priorité: 28.05.2002 FR 0206504
(43) Date de publication de la demande: 03.12.2003
(73) Titulaire: L'ORÉAL, 75008 Paris (FR)
(72) Inventeur: Rubinstenn, Gilles, 75005 Paris (FR)
(74) Mandataire: Kromer, Christophe

(56) Documents cités:
- US-A- 4 542 129

## Description

La présente invention concerne l'utilisation d'une composition renfermant au moins un dérivé 7-oxydé de DHEA pour le traitement cosmétique ou dermatologique des peaux sèches oligoséborrhéiques, ou du cuir chevelu sec.

De nombreuses femmes à partir de trente-cinq ans, et plus particulièrement après la ménopause, se plaignent fréquemment du dessèchement de leur peau, et des manifestations d'inconfort ou inesthétiques qui en résultent (desquamation, teint terne, atonie cutanée). Or, ce dessèchement est dû, comme on le sait maintenant, à une diminution de la production de sébum avec l'âge.

Par ailleurs, les enfants dont la fonction sébacée n'est pas encore active présentent souvent des signes de peau sèche.

Le sébum est le produit naturel de la glande sébacée qui, conjointement à la sueur produite par les glandes eccrines ou aprocrines, constitue un hydratant naturel de l'épiderme. Il est constitué essentiellement d'un mélange plus ou moins complexe de lipides. Classiquement, la glande sébacée produit du squalène, des triglycérides, des cires aliphatiques, des cires de cholestérol et éventuellement du cholestérol libre (Stewart, M. E., Semin. Dermatol. 11, 100-105 (1992)). L'action des lipases bactériennes convertit une part variable des triglycérides en acides gras libres.

Le sébocyte constitue la cellule compétente de la glande sébacée. La production de sébum est associée au programme de différenciation terminale de cette cellule. Durant cette différenciation, l'activité métabolique du sébocyte est essentiellement axée sur la biosynthèse des lipides (la lipogénèse) et plus précisément sur la néosyntèse d'acides gras et du squalène.

Un composé permettant de stimuler la production des lipides constituant le sébum, par les cellules de la glande sébacée (les sébocytes), serait donc d'un intérêt certain pour le traitement des peaux sèches oligoséborrhéiques, c'est-à-dire présentant un taux de sébum inférieur à 100 µg/cm² au niveau du front.

A cette fin, il a été proposé dans le brevet US-4,496,556 d'utiliser la DHEA, un stéroïde sécrété par les glandes surrénales, ou ses esters (en position 3), administrés par voie topique, pour augmenter la production dé sébum.

Toutefois, pour des questions réglementaires, il n'est pas toujours possible d'utiliser ce type de composés dans le domaine cosmétique compte tenu des effets hormonaux qu'ils sont susceptibles d'engendrer. Il subsiste donc le besoin de disposer de composés cosmétiquement acceptables, permettant de stimuler efficacement la fonction sébacée en vue de traiter les peaux sèches oligoséborrhéiques.

La Demanderesse a maintenant découvert avec étonnement que certains dérivés de DHEA oxydés en position 7 du noyau stéroïde, en particulier la 7-hydroxy DHEA, permettaient de satisfaire ce besoin, dans la mesure où ils ont un effet positif sur la production de sébum tout en étant dépourvus de l'activité hormonale de la DHEA.

La 7α-OH DHEA est un métabolite majeur de la DHEA, qui est connu pour augmenter la prolifération des fibroblastes et la viabilité des kératinocytes humains et comme agent anti-radicalaire (WO 98/40074). Il a également été mis en évidence (WO 00/28996) que la 7α-hydroxy DHEA augmentait l'épaisseur du derme et le contenu en élastine et collagène de la peau. Il a ainsi été suggéré d'utiliser ce métabolite de DHEA pour prévenir et/ou traiter les effets néfastes des UV sur la peau, lutter contre les rides et augmenter la fermeté et la tonicité de la peau. Toutefois, il n'a encore jamais été suggéré à ce jour d'utiliser la 7-hydroxy DHEA pour le traitement des peaux sèches.

La présente invention a donc pour objet l'utilisation cosmétique, pour le traitement des peaux sèches ou du cuir chevelu sec oligoséborrhéiques, d'une composition renfermant au moins un dérivé 7-oxydé de la DHEA répondant à la formule (I) suivante 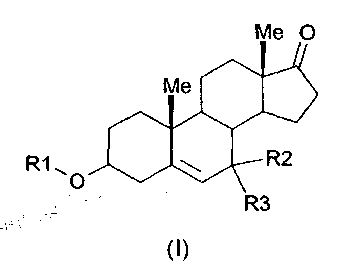 dans laquelle :
R₁ est choisi parmi un atome d'hydrogène, un groupe alkylcarbonyle et un groupe arylcarbonyle, R₂ est un atome d'hydrogène et R₃ est un radical -OR₄ où R₄ est choisi parmi un atome d'hydrogène, un groupe alkylcarbonyle et un groupe arylcarbonyle.

La présente invention a également pour objet l'utilisation cosmétique d'au moins un dérivé de DHEA tel que défini précédemment, en tant qu'agent pour le traitement des peaux sèches ou du cuir chevelu sec oligoséborrhéiques.

Les peaux sèches et cuirs chevelus secs oligoséborrhéiques visés selon l'invention sont ceux de personnes présentant un taux de sébum inférieur à 100 µg/cm² au niveau du front. Ce type de peau se rencontre fréquemment chez les femmes au voisinage de la ménopause, de sorte que la composition utilisée selon l'invention est de préférence appliquée sur des femmes de plus de quarante ans.

La présente invention a encore pour objet l'utilisation d'au moins un dérivé de DHEA tel que défini précédemment, pour la préparation d'une composition, notamment dermatologique, destinée à traiter les désordres liés aux peaux sèches oligoséborrhéiques, en particulier les dermites.

Le groupe arylcarbonyle préféré selon l'invention est un groupe benzoyle.

Parmi les groupes alkylcarbonyles, on peut citer ceux dont la partie alkyle renferme de un à six atomes de carbone et préférentiellement un groupe acétyle.

Les dérivés 7-oxydés de DHEA convenant particulièrement bien à une utilisation dans la présente invention sont la 7-hydroxy DHEA et la 3-O-acétyl 7-benzoyloxy DHEA.

Par "7-hydroxy DHEA", on entend aussi bien que 7α-OH DHEA que la 7β-OH DHEA. Un procédé de préparation de la 7α-OH DHEA est notamment décrit dans les demandes de brevet FR-2 771 105 et WO 94/08588.

La 3-O-acétyl 7-benzoyloxy DHEA est notamment disponible auprès de la société GATTEFOSSE sous la dénomination commerciale 3-acetoxy 7-benzoate DHEA.

La quantité de dérivé de DHEA utilisable selon l'invention est bien entendu fonction de l'effet recherché et peut donc varier dans une large mesure. D'une manière générale, le dérivé de DHEA sera présent en une quantité suffisante pour augmenter significativement la production de sébum et avantageusement pour augmenter d'au moins 10% la production de sébum par une culture de sébocytes, comme décrit dans l'Exemple 1 ci-après.

Pour donner un ordre de grandeur, on peut utiliser le dérivé de DHEA en une quantité représentant de 0,001% à 5% du poids total de la composition, préférentiellement en une quantité représentant de 0,05% à 1% du poids total de la composition.

La composition selon l'invention est généralement adaptée à une application topique sur la peau (y compris le cuir chevelu), de préférence la peau du visage, et elle contient donc un milieu physiologiquement acceptable, c'est-à-dire compatible avec la peau et/ou ses phanères (cils, ongles, cheveux).

Cette composition peut se présenter sous toutes les formes galéniques normalement utilisées dans les domaines cosmétique et dermatologique, et elle peut être notamment sous forme d'une solution huileuse éventuellement gélifiée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau. Elle peut aussi être utilisée comme shampooing ou après-shampooing.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des dérivés de DHEA selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents apaisants ; et les agents stimulant la prolifération et/ou la différenciation des kératinocytes.

En effet, la stimulation de la séborrhée par les dérivés de DHEA selon l'invention peut, chez certaines personnes, fournir un terrain de prolifération pour la microflore résidente de l'ostium folliculaire (*Propionibacterium acnes* en particulier), provoquant ainsi une hydrolyse importante des triglycérides du sébum en acides gras libres et la réduction des insaturations des acides gras poly-insaturés (acide linoléique en particulier). Ces deux phénomènes peuvent concourir à une kératinisation de l'infundibulum et à la formation d'un micro-comédon. Celui-ci peut dégénérer en comédon, bouchant et dilatant le pore de façon inesthétique. A un stade plus avancé, ce bouchon peut évoluer vers une lésion acnéique inflammatoire.

L'ajout d'agents desquamants ou régulant la prolifération ou la différenciation des kératinocytes à la composition selon l'invention permettent d'éviter la formation de ces comédons. De même, des agents anti-bactériens ou bactério-statiques permettraient, en modérant la prolifération de la microflore résidente, d'obtenir le même effet.

En outre, les agents hydratants peuvent compléter l'effet obtenu à l'aide des dérivés de DHEA selon l'invention, et les agents apaisants sont utiles pour améliorer le confort des peaux sèches oligoséborrhéiques.

L'invention sera maintenant illustrée par les exemples non limitatifs suivants. Dans ces exemples, les quantités sont indiquées en pourcentage pondéral.

### EXEMPLES

### Exemple 1 : Mise en évidence de l'activité des dérivés 7-oxydés de DHEA sur la lipogénèse

La 7α-OH DHEA et la 3-O-acétyl 7-benzoyloxy DHEA ont été testées sur un modèle de sébocytes humains immortalisés en culture, issus de la lignée SZ95 décrite dans Zouboulis, C.C., Seltmann, H., Neitzel, H. & Orfanos, C.E., Establishment and Characterization of an Immortalized Human Sebaceous Gland Cell Line, J. Invest. Dermatol., 113, 1011-1020 (1999).

Le test a consisté à mesurer la quantité de lipides produite par les sébocytes de la lignée (à confluence), en présence ou non d'agents actifs dilués dans le DMSO à 10⁻⁵ M et 10⁻⁶ M, de telle sorte que la quantité finale de DMSO dans le milieu de culture soit de 0,1%. Après 2 jours de traitement, les cellules adhérentes sont traitées par du Rouge de Nile (1µg/ml). Le contenu en lipides est ensuite quantifié par mesure de la fluorescence du colorant (deux couples d'excitation/émission : 485-540nm pour les lipides neutres et 540-620 nm pour les lipides non neutres). Les résultats sont donnés pour les lipides totaux (combinaison des deux mesures).

L'expérience est réalisée en sixplicate (produits dosés) ou dodeplicate (témoin) en plaque de 96 puits.

Le Tableau ci-dessous rassemble les résultats obtenus, en pourcentage de variation par rapport au témoin, ainsi que les coefficients p fournis par le test de Student et qui reflètent la significativité des mesures.

| | % de variation à 10⁻⁶ M | p | % de variation à 10⁻⁵ M | p |
|---|---|---|---|---|
| 7α-OH DHEA | + 6 % | 0,217 (ns) | + 15 % | 0,003 |
| 3-O-acétyl 7-benzoyloxy DHEA | + 15 % | 0,012 | + 15 % | 0,001 |

Comme il ressort de ce tableau, les deux dérivés 7-oxydés de DHEA entraînent une augmentation significative de la la lipogénèse à 10⁻⁵M et la 3-O-acétyl 7-benzoyloxy DHEAest également efficace à 10⁻⁶ M.

En outre, il a été vérifié qu'aucun des deux composés testés ne présentait d'effet sur la production des lipides polaires (non spécifiques du sébum), et n'induisait de mortalité cellulaire (test LDH) ni de variation de la prolifération cellulaire (test au MUH).

### Exemple 2 : Compositions cosmétiques et dermatologiques

Ces compositions sont préparées de manière classique pour l'homme du métier. Les quantités données dans ces exemples sont indiquées en pourcentages pondéraux.

### A. Lotion

| | |
|---|---|
| 7α-OH DHEA | 1 % |
| Acide salicylique | 1 % |
| Propylène glycol | 5 % |
| Alcool | 87 % |
| Eau | qsp 100 % |

Cette lotion peut être utilisée le soir pour redynamiser la fonction sébacée.

### B. Crème

| | |
|---|---|
| 3-O-acétyl 7-benzoyloxy DHEA | 1 % |
| Acide n-octanoyl-5-salicylique | 1 % |
| Méthylparaben | 0.1 % |
| Propylparaben | 0.1 % |
| Lanoline | 5 % |
| Huile de vaseline | 4 % |
| Huile de sésame | 4 % |
| Alcool cétylique | 5 % |
| Monostéarate de glycérol | 2 % |
| Triéthanolamine | 1 % |
| Propylène glycol | 5 % |
| Carbomer 940 | 0.1 % |
| Eau | qsp 100 % |

Cette crème, utilisée en applications bi-quotidiennes, permet de raviver l'éclat des peaux sèches.

### C. Onguent

| | |
|---|---|
| 7α-OH DHEA | 1 % |
| Acide salicylique | 1 % |
| Monostéarate de glycérol | 3 % |
| Propylène glycol | 12 % |
| Petrolatum | 82.9 % |
| Eau | qsp 100 % |

### D. Gel

| | |
|---|---|
| 3-O-acétyl 7-benzoyloxy DHEA | 1 % |
| Acide salicylique | 1 % |
| Hydroxy propyl cellulose | 1 % |
| PPG-12-Buteth-16 | 2 % |
| Triéthanolamine | 0.2 % |
| Propylène glycol | 5 % |
| Alcool | 45 % |
| Carbomer 940 | 0.2 % |
| Eau | qsp 100 % |

### E. Emulsion huile dans eau

| | |
|---|---|
| 3-O-acétyl 7-benzoyloxy DHEA | 0.3 % |
| Extrait lyophylisé de romarin | 0.2 % |
| Stéarate de glycérol | 2 % |
| Polysorbate 60 | 1 % |
| Acide stéarique | 1.4 % |
| Triéthanolamine | 0.7 % |
| Carbomer | 0.4 % |
| Huile d'olive | 12 % |
| Fraction liquide du beurre de karité | 12 % |
| Octyldodécanol | 6 % |
| Isononanoate d'isononyle | 10 % |
| Antioxydant | 0.05 % |
| Parfum | 0.5 % |
| Conservateur | 0.3 % |
| Eau | qsp 100 % |

### F. Crème

| | |
|---|---|
| 7β-OH DHEA | 0.5 % |
| Rétinol | 0.1 % |
| Glycérine | 3 % |
| Gomme de Xanthane | 0.1 % |
| Stéarate de sorbitan oxyéthyléné | 0.9 % |
| PEG-100 stéarate et glycéryl stéarate | 2.1 % |
| Alcool cétylique | 2.60 % |
| Isononanoate d'isononyle | 11 % |
| Octydodécanol | 15 % |
| Butylhydroxytoluène | 0.1 % |
| Octocrylène | 2 % |
| Triéthanolamine | 0.3 % |
| Acétate de tocophérol | 1 % |
| Conservateurs | 0.6 % |
| Eau | qsp 100 % |

### G. Crème

| | |
|---|---|
| 3-O-acétyl 7-benzoyloxy DHEA | 0.3 % |
| O-octanoyl-6'-D-maltose * | 2 % |
| Triéthanolamine | 0.3 % |
| PEG-100 stéarate et glycéryl stéarate | 2.5 % |
| PEG-50 stearate | 2.5 % |
| Alcool cétylique | 1 % |
| Alcool stéarylique | 3 % |
| lsononaoate d'isononyle | 20 % |
| Propylparaben | 0.1 % |
| Carbopol | 0.3 % |
| Eau | qsp 100 % |

| | |
|---|---|
| * obtenu comme décrit dans la demande EP-0 566 438 | |

## Revendications

1. Utilisation cosmétique, pour le traitement des peaux sèches ou du cuir chevelu sec oligoséborrhéiques, d'une composition renfermant au moins un dérivé 7-oxydé de la DHEA répondant à la formule (I) suivante : 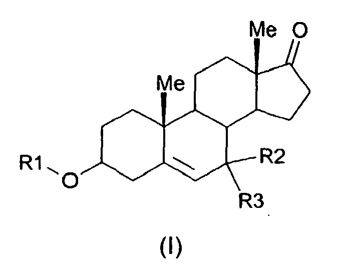 dans laquelle :
R₁ est choisi parmi un atome d'hydrogène, un groupe alkylcarbonyle et un groupe arylcarbonyle, R₂ est un atome d'hydrogène et R₃ est un radical -OR₄ où R₄ est choisi parmi un atome d'hydrogène, un groupe alkylcarbonyle et un groupe arylcarbonyle.

2. Utilisation cosmétique d'au moins un dérivé de DHEA tel que défini dans la revendication 1, en tant qu'agent pour le traitement des peaux sèches ou du cuir chevelu sec oligoséborrhéiques.

3. Utilisation d'au moins un dérivé de DHEA tel que défini dans la revendication 1, pour la préparation d'une composition destinée à traiter les désordres liés aux peaux sèches oligoséborrhéiques.

4. Utilisation selon la revendication 3, **caractérisée en ce que** lesdits désordres sont les dermites.

5. Utilisation selon l'une quelconque des revendications 1 à 4, **caractérisée en ce que** ladite composition est appliquée sur des femmes de plus de quarante ans.

6. Utilisation selon l'une quelconque des revendications 1 à 5, **caractérisée en ce que** ledit groupe arylcarbonyle est un groupe benzoyle.

7. Utilisation selon l'une quelconque des revendications 1 à 5, **caractérisée en ce que** ledit groupe alkylcarbonyle est tel que sa partie alkyle renferme de un à six atomes de carbone.

8. Utilisation selon la revendication 7, **caractérisée en ce que** ledit groupe alkylcarbonyle est un groupe acétyle.

9. Utilisation selon l'une quelconque des revendications 1 à 5, **caractérisée en ce que** ledit dérivé de DHEA est la 7-hydroxy DHEA.

10. Utilisation selon l'une quelconque des revendications 1 à 6, **caractérisée en ce que** ledit dérivé de DHEA est la 3-O-acétyl 7-benzoyloxy DHEA.

11. Utilisation selon l'une quelconque des revendications précédentes, **caractérisée en ce que** ledit dérivé de DHEA représente de 0,05% à 1% du poids total de la composition.

12. Utilisation selon l'une quelconque des revendications précédentes, **caractérisée en ce que** la composition est adaptée à une application topique sur la peau.

13. Utilisation selon l'une quelconque des revendications précédentes, **caractérisée en ce que** ladite composition renferme en outre au moins un composé choisi parmi : les agents desquamants ; les agents hydratants ; les agents apaisants ; et les agents stimulant la prolifération et/ou la différenciation des kératinocytes.

14. Utilisation selon l'une quelconque des revendications précédentes, **caractérisée en ce que** la composition est sous la forme d'une émulsion huile-dans-eau.

## Claims

1. Cosmetic use, for treating oligoseborrhoeic dry skin or an oligoseborrhoeic dry scalp, of a composition containing at least one 7-oxido DHEA derivative corresponding to formula (I) below: 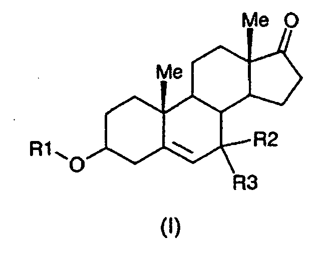 in which:
R₁ is chosen from a hydrogen atom, an alkylcarbonyl group and an arylcarbonyl group, R₂ is a hydrogen atom and R₃ is a radical -OR₄ in which R₄ is chosen from a hydrogen atom, an alkylcarbonyl group and an arylcarbonyl group.

2. Cosmetic use of at least one DHEA derivative as defined in Claim 1, as an agent for treating oligoseborrhoeic dry skin or an oligoseborrhoeic dry scalp.

3. Use of at least one DHEA derivative as defined in Claim 1, for the preparation of a composition for treating disorders associated with oligoseborrhoeic dry skin.

4. Use according to Claim 3, **characterized in that** the said disorder is dermatitis.

5. Use according to any one of Claims 1 to 4, **characterized in that** the said composition is applied to women over forty years old.

6. Use according to any one of Claims 1 to 5, **characterized in that** the said arylcarbonyl group is a benzoyl group.

7. Use according to any one of Claims 1 to 5, **characterized in that** the said alkylcarbonyl group is such that its alkyl portion contains from one to six carbon atoms.

8. Use according to Claim 7, **characterized in that** the said alkylcarbonyl group is an acetyl group.

9. Use according to any one of Claims 1 to 5, **characterized in that** the said DHEA derivative is 7-hydroxy DHEA.

10. Use according to any one of Claims 1 to 6, **characterized in that** the said DHEA derivative is 3-O-acetyl 7-benzoyloxy DHEA.

11. Use according to any one of the preceding claims, **characterized in that** the said DHEA derivative represents from 0.05% to 1% of the total weight of the composition.

12. Use according to any one of the preceding claims, **characterized in that** the composition is suitable for topical application to the skin.

13. Use according to any one of the preceding claims, **characterized in that** the said composition also contains at least one compound chosen from desquamating agents; moisturizers; calmants; and agents for promoting keratinocyte proliferation and/or differentiation.

14. Use according to any one of the preceding claims, **characterized in that** the composition is in the form of an oil-in-water emulsion.

## Patentansprüche

1. Kosmetische Verwendung einer Zusammensetzung, die mindestens ein 7-oxidiertes Derivat von DHEA der folgenden Formel (I) enthält, zur Behandlung von oligoseborrhoeischer trockener Haut oder Kopfhaut: 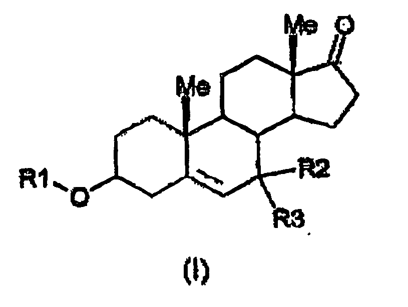 worin bedeuten:
R₁ ist unter einem Wasserstoffatom, einer Alkylcarbonylgruppe und einer Arylcarbonylgruppe ausgewählt, R₂ ist ein Wasserstoffatom und R₃ ist eine Gruppe -OR₄, wobei R₄ unter einem Wasserstoffatom, einer Alkylcarbonylgruppe oder einer Arylcarbonylgruppe ausgewählt ist.

2. Kosmetische Verwendung mindestens eines DHEA-Derivats nach Anspruch 1 als Wirkstoff zur Behandlung von oligoseborrhoeischer trockener Haut oder oligoseborrhoeischer trockener Kopfhaut.

3. Verwendung mindestens eines DHEA-Derivats nach Anspruch 1 zur Herstellung einer Zusammensetzung, die zur Behandlung von Störungen vorgesehen ist, die mit oligoseborrhoeischer trockener Haut zusammenhängen.

4. Verwendung nach Anspruch 3, **dadurch gekennzeichnet, dass** es sich bei den Störungen um Dermatitis handelt.

5. Verwendung nach einem der Ansprüche 1 bis 4, **dadurch gekennzeichnet, dass** die Zusammensetzung bei Frauen mit einem Alter über 40 Jahren aufgetragen wird.

6. Verwendung nach einem der Ansprüche 1 bis 5, **dadurch gekennzeichnet, dass** die Arylcarbonylgruppe eine Benzoylgruppe ist.

7. Verwendung nach einem der Ansprüche 1 bis 5, **dadurch gekennzeichnet, dass** die Alkylcarbonylgruppe so vorliegt, dass der Alkylteil ein bis sechs Kohlenstoffatome enthält.

8. Verwendung nach Anspruch 7, **dadurch gekennzeichnet, dass** die Alkylcarbonylgruppe eine Acetylgruppe ist.

9. Verwendung nach einem der Ansprüche 1 bis 5, **dadurch gekennzeichnet, dass** das DHEA-Derivat das 7-Hydroxy-DHEA ist.

10. Verwendung nach einem der Ansprüche 1 bis 6, **dadurch gekennzeichnet, dass** das DHEA-Derivat 3-0-Acetyl-7-benzoyloxy-DHEA ist.

11. Verwendung nach einem der vorhergehenden Ansprüche, **dadurch gekennzeichnet, dass** das DHEA-Derivat 0,05 bis 1 % des Gesamtgewichts der Zusammensetzung ausmacht.

12. Verwendung nach einem der vorhergehenden Ansprüche, **dadurch gekennzeichnet, dass** die Zusammensetzung für eine topische Anwendung auf die Haut geeignet ist.

13. Verwendung nach einem der vorhergehenden Ansprüche, **dadurch gekennzeichnet, dass** die Zusammensetzung ferner mindestens eine Verbindung enthält, die ausgewählt ist unter: abschuppenden Wirkstoffen; Hydratisierungsmitteln; beruhigenden Wirkstoffen; und Wirkstoffen, die die Proliferation und/oder die Differenzierung der Keratinocyten stimulieren.

14. Verwendung nach einem der vorhergehenden Ansprüche, **dadurch gekennzeichnet, dass** die Zusammensetzung als Öl-in-Wasser-Emulsion vorliegt.
